# FASCICULE DE BREVET EUROPEEN

(11) **EP 1 328 239 B1**
(45) Date de publication et mention de la délivrance du brevet: **16.02.2005**
(21) Numéro de dépôt: 01982524.9
(22) Date de dépôt: 19.10.2001
(51) Int. Cl.: A61K 7/027

(54) **PATE PIGMENTAIRE ANHYDRE ET SON UTILISATION EN COSMETIQUE**
WASSERFREIE PIGMENTPASTE UND IHRE ANWENDUNG IN DER KOSMETIK
ANHYDROUS PIGMENT PASTE AND ITS USE IN COSMETICS

(30) Priorité: 19.10.2000 FR 0013399
(43) Date de publication de la demande: 23.07.2003
(73) Titulaire: L'OREAL, 75008 Paris (FR)
(72) Inventeur: TOURNILHAC, Florence, F-75011 PARIS (FR); KLUCKER, Rolf, F-69004 Lyon France (FR)
(74) Mandataire: Catherine, Alain
(86) Numéro de dépôt international: PCT/FR2001/003260
(87) Numéro de publication internationale: WO 2002/032382

(56) Documents cités:
- FR-A- 2 772 605
- US-A- 3 406 238

## Description

La présente invention se rapporte à une pâte pigmentaire anhydre pour teinter des compositions cosmétiques, comprenant des pigments solides, dispersés dans un milieu continu par un agent dispersant.

Par pâte pigmentaire, on entend une dispersion colloïdale concentrée de pigments dans un milieu continu, ou milieu de dispersion, stabilisée à l'aide d'un agent dispersant ou sans agent dispersant.

On entend par dispersion colloïdale concentrée une suspension de particules de taille micronique, c'est-à-dire inférieure à 10 µm, dans un milieu continu. La fraction volumique de particules dans une dispersion concentrée est de l'ordre de 20 à 40%, de préférence supérieure à 30%. Tout liquide souhaité pour la dispersion peut tenir lieu de milieu continu, par exemple l'eau, le glycol, les solvants, les matières grasses et leurs mélanges.

L'agent dispersant protège les particules dispersées contre l'agglomération ou la floculation. L'agent dispersant peut être un tensioactif, un oligomère, un polymère ou un mélange de plusieurs d'entre eux, portant une ou des fonctionnalités ayant une affinité forte pour la surface de la particule à disperser.

Ces pigments sont difficiles à disperser dans les huiles hydrocarbonées ou les huiles siliconées, classiquement utilisées dans les rouges à lèvres, et ont tendance à s'y agglomérer, même en présence d'agents dispersants classiques. Cela limite la concentration maximale en pigments dans l'huile, et nuit à la fluidité d'une telle dispersion.

L'utilisation d'agents dispersants dans des pâtes pigmentaires est connue dans l'art antérieur. Cependant, les pâtes pigmentaires contenant ces mêmes pigments organiques en présence d'un agent dispersant classique sont relativement épaisses et présentent une viscosité élevée, traduisant une mauvaise affinité d'adsorption de l'agent dispersant pour la surface des pigments organiques.

L'invention a donc pour objet une pâte pigmentaire qui remédie aux inconvénients de l'art antérieur.

En particulier, l'invention a pour objet une pâte pigmentaire anhydre comprenant un agent dispersant montrant une bonne affinité d'adsorption pour la surface des pigments, notamment celle des pigments organiques. La présence d'un tel agent présente l'avantage de pouvoir fabriquer des pâtes pigmentaires ayant une faible viscosité, tout en maintenant une concentration élevée en pigments.

L'invention a également pour objet l'utilisation d'une pâte pigmentaire anhydre selon l'invention pour teinter des compositions cosmétiques, et notamment des rouges à lèvres.

L'invention a encore pour objet une composition cosmétique contenant un milieu cosmétiquement acceptable et une pâte pigmentaire selon l'invention.

Par cosmétiquement acceptable, on entend, au sens de la demande, une composition d'aspect, d'odeur, de goût et de toucher agréable.

Les buts de la présente invention sont atteints en réalisant une pâte pigmentaire anhydre comprenant:
· de 20 à 32% en volume d'au moins un pigment solide, par rapport au volume total de la pâte pigmentaire,
· un milieu de dispersion des pigments comprenant au moins un solvant anhydre choisi parmi les huiles hydrocarbonées et les huiles siliconées, et
· un agent dispersant qui est un polymère comprenant au moins un groupement imide ou succinimide lié par une liaison covalente à une chaîne polymère compatible avec le milieu de dispersion.

Le ou les groupements imide ou succinimide peuvent être des groupements latéraux ou terminaux, de préférence terminaux.

Par chaîne polymère compatible, on entend une chaîne polymère ou oligomère soluble dans la phase continue de la dispersion.

De manière avantageuse, la chaîne polymère a un poids moléculaire d'au moins 600 g, et de préférence allant de 1000 à 1500 g.

Les chaînes polymères préférées selon l'invention sont les polyoléfines, telles que les polyéthylènes, les polypropylènes et les polyisobutylènes et autres poly α-oléfines. Les chaînes polymères particulièrement préférées sont des polyisobutylènes.

Les agents dispersants utilisables selon l'invention sont de préférence des polymères obtenus par condensation d'une amine sur un anhydride succinique ayant une chaîne hydrocarbonée saturée ou non saturée, dont.la synthèse est décrite dans le brevet US 3,172,892. Les agents dispersants préférés sont des polyisobutylènes comprenant au moins un groupement succinimide, de préférence un groupement terminal succinimide. Ces agents dispersants ont avantageusement une chaîne polymère de poids moléculaire d'au moins 600 g, et mieux de poids moléculaire allant de 1000 à 1500 g. De tels agents dispersants sont décrits dans les brevets US-3,172,892 et 3,235,484, et sont connus sous le nom de LUBRIZOL. En particulier, un agent dispersant préféré est le polyisobutylène succinimide commercialisé sous la dénomination commerciale LUBRIZOL 0517134.

En général, l'agent dispersant représente de 1 à 10% de la masse totale de la pâte pigmentaire, et de préférence de l'ordre de 5%.

La pâte pigmentaire selon l'invention contient également au moins un pigment habituellement utilisé dans les compositions cosmétiques ou dermatologiques.

Les pigments utilisables selon l'invention peuvent être des pigments minéraux ou organiques, blancs ou colorés, ou nacrés, ou leurs mélanges.

On peut citer, parmi les pigments minéraux, le dioxyde de titane, éventuellement traité en surface, ou de zinc, les oxydes de zirconium ou de cérium, ainsi que les oxydes de fer ou de chrome, le violet de manganèse, le bleu outremer, l'hydrate de chrome et le bleu ferrique, et leurs mélanges. Les pigments minéraux préférés sont les oxydes de fer, notamment l'oxyde de fer rouge, l'oxyde de fer jaune, l'oxyde de fer brun, l'oxyde de fer noir, le dioxyde de titane, et leurs mélanges.

Parmi les pigments organiques, on peut citer :
- les pigments organiques purs, tel le noir de carbone ou ceux qui sont soumis ou non à une certification de la FDA, tel que le D&C Red No. 36, et
- les pigments de type laque organique tels que les laques organiques de baryum, de strontium, de calcium ou d'aluminium soumis à une certification par la FDA ou exempts de certification par la FDA comme les laques à base de carmin de cochenille, le D&C Red N° 7 calcium lake, le D&C Red N° 27 aluminium lake, le D&C Red No. 21 aluminium lake, le FD&C Yellow N°5 aluminium lake, le FD&C Yellow No. 6 aluminium lake, le D&C Red No. 7 et le FD&C Blue No. 1 aluminium lake, et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité.

L'agent dispersant selon l'invention montre une bonne affinité d'adsorption pour la surface des pigments, et en particulier les pigments organiques.

Le milieu de dispersion des pigments solides contient au moins un solvant anhydre choisi parmi les huiles hydrocarbonées et les huiles siliconées. Ces huiles peuvent être des huiles polaires ou non polaires, volatiles ou non volatiles, utilisables en cosmétique.

Parmi les huiles polaires, on peut citer les huiles hydrocarbonées comportant des fonctions esters, éthers, acides, alcools ou leurs mélanges, telles que par exemple :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat; ou encore les triglycérides des acides caprylique/caprique comme ceux vendus par la société Stearineries Dubois ou ceux vendus sous les dénominations Miglyol( 810, 812 et 818 par la société Dynamit Nobel;
- les huiles de synthèse de formule R₁COOR₂ dans laquelle R₁ représente le reste d'un acide gras supérieur, linéaire ou, ramifié, comportant de 7 à 19 atomes de carbone, et R₂ représente une chaîne hydrocarbonée ramifiée contenant de 3 à 20 atomes de carbone, comme par exemple l'huile de Purcellin (octanoate de cétostéaryle), l'isononanoate d'isononyle, le benzoate d'alcool en C₁₂ à C₁₅;
- les esters et les éthers de synthèse comme le myristate d'isopropyle, le palmitate d'éthyl-2-hexyle, les octanoates, les décanoates ou les ricinoléates d'alcools ou de polyalcools,
- les esters hydroxylés comme le lactate d'isostéaryle, le malate de di-isostéaryle, et les esters du pentaérythritol;
- les alcools gras en C₈ à C₂₆ comme l'alcool oléique; et
- leurs mélanges.

De préférence, le solvant anhydre est choisi parmi les huiles apolaires.

Parmi les huiles apolaires, on peut citer :
- les huiles de silicone volatiles ou non, linéaires ou cycliques, liquides à température ambiante, telles que les polydiméthylsiloxanes (PDMS) comportant des groupements alkyle, alcoxy ou phényle, pendants et/ou en bout de chaîne siliconée et ayant de 2 à 24 atomes de carbone; les silicones phénylées, comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthyisiloxysilicates, et leurs mélanges.
- les hydrocarbures ou les hydrocarbures fluorés, linéaires ou ramifiés d'origine synthétique ou minérale, telles que les huiles de paraffine (par exemple les isoparaffines), et les hydrocarbures aliphatiques volatiles (par exemple l'isododécane), ou non volatiles et leurs dérivés, la vaseline, les polydécènes, le polyisobutène hydrogéné tel que le Parléam® (polyisobutène hydrogéné), le squalane, et leurs mélanges.

De préférence, le solvant anhydre est une huile hydrocarbonée apolaire, d'origine minérale ou synthétique, choisie notamment parmi les hydrocarbures, notamment les alcanes, comme le Parléam® (polyisobutène hydrogéné), les isoparaffines dont l'isododécane, le squalane, et leurs mélanges. Le solvant anhydre particulièrement préféré est le Parléam® (polyisobutène hydrogéné).

Les pâtes pigmentaires selon l'invention ont généralement une viscosité de 2 à 25 Poises (0,2 à 2,5 Pa.s), de préférence de 10 Poises (1 Pa.s) ou moins, et mieux de 5 Poises (0,5 Pa.s) ou moins, la viscosité étant évaluée à température ambiante (20 à 25°C), avec un taux de cisaillement de 200 s⁻¹, à l'aide un viscosimètre Rhéomat RM 180 avec les mobiles 2 ou 3 selon leur adéquation à la viscosité de la pâte pigmentaire.

Les pâtes pigmentaires selon l'invention conviennent particulièrement pour teinter des compositions cosmétiques, et en particulier dans la formulation des rouges à lèvres.

Les exemples suivants illustrent l'invention sans toutefois en limiter la portée.

Dans les exemples, sauf indication contraire, toutes les quantités sont exprimées en fraction massique par rapport au poids total de la pâte pigmentaire.

### EXEMPLES

On a préparé les pâtes pigmentaires PP1 à PP5 selon l'invention en mélangeant les ingrédients indiqués dans le tableau 1 ci-après comme suit :
1. l'agent dispersant est dissous dans l'huile de parléam, à une température d'environ 80°C, pendant une durée de 10 à 15 minutes, afin d'assurer la dissolution complète de l'agent dispersant;
2. les pigments sont ensuite progressivement ajoutés à l'huile de parléam sous agitation par un disque défloculateur pour assurer le mouillage complet des pigments, puis le mélange est homogénéisé pendant d'environ 15 minutes;
3. ce mélange est broyé dans un broyeur DISPERMAT pendant une durée de 35 à 40 minutes, à une température de 25 à 30°C.

Pour chaque pâte pigmentaire, PP1 à PP5, on mesure la viscosité à température ambiante (20 à 25°C) et un taux de cisaillement de 200 s⁻¹, avec un viscosimètre RHEOMAT RM 180 avec les mobiles 2 ou 3 selon leur adéquation à la viscosité de la pâte pigmentaire.

Les résultats des mesures de viscosité des pâtes pigmentaires PP1 à PP5 sont donnés dans le tableau 2.

**TABLEAU 1**

| composition (%) | PP1 | PP2 | PP3 | PP4 | PP5 |
|---|---|---|---|---|---|
| D&C Red No 36 | 40% | 40% | - | - | - |
| D&C Red No 7 | - | (26,1% en volume) | 30% | 30% | 40% |
| huile de parléam | 57% | 55% | 67% | 65% | 55% |
| LUBRIZOL OS17134AZ | 3% | 5% | 3% | 5% | 5% |

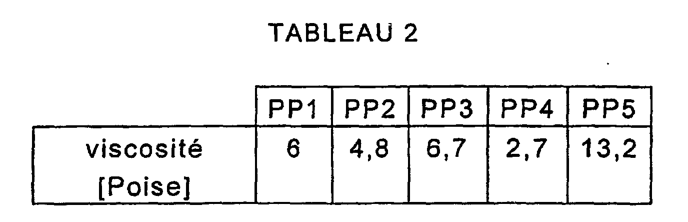

Le tableau 2 montre que les pâtes pigmentaires selon l'invention PP1 à PP5 ont une viscosité faible, c'est à dire inférieure à 15 Poises, pour une fraction massique de pigment élevée.

Le tableau 2 montre également que, pour une même quantité de pigment, une augmentation de la fraction massique d'agent dispersant entraîne une diminution supplémentaire de la viscosité.

## Revendications

1. Pâte pigmentaire anhydre comprenant :
• de 20 à 32 % en volume d'au moins un pigment solide, par rapport au volume total de la pâte pigmentaire,
• un milieu de dispersion du pigment comprenant au moins un solvant anhydre choisi parmi les huiles hydrocarbonées et les huiles siliconées, et
• un agent dispersant qui est un polymère comprenant au moins un groupement imide ou succinimide lié par une liaison covalente à une chaîne polymère compatible avec le milieu de dispersion.

2. Pâte pigmentaire selon la revendication 1, **caractérisée en ce que** la chaîne polymère a un poids moléculaire d'au moins 600 g.

3. Pâte pigmentaire selon la revendication 1 ou 2, **caractérisée en ce que** la chaîne polymère a un poids moléculaire allant de 1000 à 1500 g.

4. Pâte pigmentaire selon l'une quelconque des revendications précédentes, **caractérisée en ce que** la chaîne polymère est une polyoléfine.

5. Pâte pigmentaire selon l'une quelconque des revendications précédentes, **caractérisée en ce que** la chaîne polymère est un polyisobutylène.

6. Pâte pigmentaire selon l'une quelconque des revendications précédentes, **caractérisée en ce que** l'agent dispersant est un polyisobutylène succinimide.

7. Pâte pigmentaire selon l'une quelconque des revendications précédentes, **caractérisée en ce que** l'agent dispersant est un polymère obtenu par condensation d'une amine sur un anhydride succinique ayant une chaîne hycrocarbonée saturée ou non saturée.

8. Pâte pigmentaire selon l'une quelconque des revendications précédentes, **caractérisée en ce que** la fraction massique d'agent dispersant dans la pâte pigmentaire est de 1 à 10% de la masse totale de la pâte pigmentaire, et de préférence de l'ordre de 5%.

9. Pâte pigmentaire selon l'une quelconque des revendications précédentes, **caractérisée en ce que** le pigment est choisi parmi les pigments minéraux, les pigments organiques, les pigments nacrés, et leurs mélanges.

10. Pâte pigmentaire selon la revendication 9, **caractérisée en ce que** les pigments minéraux sont choisis parmi le dioxyde de titane, le dioxyde de zinc, l'oxyde de fer rouge, l'oxyde de fer jaune, l'oxyde de fer brun, l'oxyde de fer noir, et leurs mélanges.

11. Pâte pigmentaire selon la revendication 9, **caractérisée en ce que** les pigments organiques sont choisis parmi les pigments organiques purs ou de type laque.

12. Pâte pigmentaire selon l'une des revendications 9 ou 11, **caractérisée en ce que** le pigment est le pigment organique D&C Red No. 36.

13. Pâte pigmentaire selon la revendication 9, **caractérisée en ce que** le pigment est une laque choisie parmi les laques organiques de baryum, de strontium, de calcium ou d'aluminium, les laques à base de carmin de cochenille, le D&C Red N° 7 calcium lake, le D&C Red N° 27 aluminium lake, le D&C Red No. 21 aluminium lake, le FD&C Yellow N°5 aluminium lake, le FD&C Yellow No. 6 aluminium lake, le D&C Red No. 7 et le FD&C Blue No. 1 aluminium lake, et leurs mélanges.

14. Pâte pigmentaire selon la revendication 9, **caractérisée en ce que** les pigments nacrés sont choisis parmi le mica recouvert de titane ou d'oxychlorure de bismuth, le mica titane avec des oxydes de fer, le mica titane avec du bleu ferrique, le mica titane avec de l'oxyde de chrome, et le mica titane avec un pigment organique tel que défini selon l'une des revendications 11 à 13.

15. Pâte pigmentaire selon l'une quelconque des revendications précédentes, **caractérisée en ce que** le solvant anhydre est choisi parmi les huiles apolaires.

16. Pâte pigmentaire selon l'une quelconque des revendications précédentes, **caractérisée en ce que** le solvant anhydre est une huile hydrocarbonée apolaire, choisie parmi le polyisobutène hydrogéné, le squalane, les hydrocarbures aliphatiques et leurs mélanges.

17. Pâte pigmentaire selon l'une quelconque des revendications précédentes, **caractérisée en ce que** le solvant anhydre est une huile de silicone choisie parmi les polydiméthylsiloxanes (PDMS) et les silicones phénylées telles que les phényl triméthicones, les phényl diméthicones, les diphényl diméthicones, les diphényl méthyldiphényl trisiloxanes, et les 2-phényléthyl triméthylsiloxysilicates, et leurs mélanges.

18. Pâte pigmentaire selon l'une quelconque des revendications précédentes **caractérisée en ce qu'**elle a une viscosité de 2 à 25 Poises (0,2 à 2,5 Pa.s), de préférence de 10 Poises (1 Pa.s) ou moins, et mieux de 5 poises (0,5 Pa.s) ou moins, ladite viscosité étant évaluée à température ambiante (20-25°C), avec un taux de cisaillement de 200 s⁻¹.

19. Composition cosmétique contenant un milieu cosmétiquement acceptable et une pâte pigmentaire telle que définie selon l'une des revendications précédentes.

20. Utilisation de la pâte pigmentaire telle que définie selon l'une des revendications 1 à 18 pour la fabrication de rouges à lèvres

## Claims

1. Anhydrous pigment paste comprising:
• from 20 to 32% by volume of at least one solid pigment, based on the total volume of pigment paste,
• a pigment dispersion medium comprising at least one anhydrous solvent selected from the hydrocarbon oils and the silicone oils, and
• a dispersing agent which is a polymer comprising at least one imide or succinimide group linked by a covalent bond to a polymer chain compatible with the dispersion medium.

2. Pigment paste according to claim 1, **characterized in that** the polymer chain has a molecular weight of at least 600 g

3. Pigment paste according to claim 1 or 2, **characterized in that** the polymer chain has a molecular weight of from 1000 to 1500 g.

4. Pigment paste according to any one of the preceding claims, **characterized in that** the polymer chain is a polyolefin.

5. Pigment paste according to any one of the preceding claims, **characterized in that** the polymer chain is a polyisobutylene.

6. Pigment paste according to any one of the preceding claims, **characterized in that** the dispersing agent is a polyisobutylene succinimide.

7. Pigment paste according to any one of the preceding claims, **characterized in that** the dispersing agent is a polymer obtained by condensing an amine with a succinic anhydride having a saturated or unsaturated hydrocarbon chain.

8. Pigment paste according to any one of the preceding claims, **characterized in that** the weight fraction of the dispersing agent in the pigment paste is from 1 to 10% of the total weight of the pigment paste, and preferably of about 5%.

9. Pigment paste according to any one of the preceding claims, **characterized in that** the pigment is selected from the inorganic pigments, the organic pigments, the nacre pigments, and mixtures thereof.

10. Pigment paste according to claim 9, **characterized in that** the inorganic pigments are selected from titanium dioxide, zinc dioxide, red iron oxide, yellow iron oxide, brown iron oxide, black iron oxide, and mixtures thereof.

11. Pigment paste according to claim 9, **characterized in that** the organic pigments are selected from the pure organic pigments or the pigments of the lacquer type.

12. Pigment paste according to one of claims 9 or 11, **characterized in that** the pigment is the organic pigment D&C Red No 36.

13. Pigment paste according to claim 9, **characterized in that** the pigment is a lacquer selected from the organic lacquers of barium, strontium, calcium or aluminium, the lacquers based on cochineal carmine, D&C Red No 7 calcium lake, D&C Red No 27 aluminium lake, D&C Red No 21 aluminium lake, FD&C Yellow No 5 aluminium lake, FD&C Yellow No 6 aluminium lake, D&C Red No 7 and FD&C Blue No 1 aluminium lake, and mixtures thereof.

14. Pigment paste according to claim 9, **characterised in that** the nacre pigments are selected from mica coated with titanium or bismuth oxychloride, titanium mica with iron oxides, titanium mica with ferric blue, titanium mica with chromium oxide, and titanium mica with an organic pigment as defined in one of claims 11 to 13.

15. Pigment paste according to any one of the preceding claims, **characterized in that** the anhydrous solvent is selected from the apolar oils.

16. Pigment paste according to any one of the preceding claims, **characterized in that** the anhydrous solvent is an apolar hydrocarbon oil, selected from hydrogenated polyisobutene, squalane, the aliphatic hydrocarbons and mixtures thereof.

17. Pigment paste according to any one of the preceding claims, **characterized in that** the anhydrous solvent is a silicone oil selected from the polydimethylsiloxanes (PDMS) and the phenylated silicones, such as the phenyl trimethicones, the phenyl dimethicones, the diphenyl dimethicones, the diphenyl methyldiphenyl trisiloxanes, and the 2-phenylethyl trimethylsiloxysilicates, and mixtures thereof.

18. Pigment paste according to any one of the preceding claims, **characterized in that** it has a viscosity of 2 to 25 Poises (0.2 to 2.5 Pa.s), preferably of 10 Poises (1 Pa.s) or less, and more preferably of 5 poises (0.5 Pa.s) or less, said viscosity being evaluated at room temperature (20-25°C), with a shearing rate of 200 s⁻¹.

19. Cosmetic composition comprising a cosmetically acceptable medium and a pigment paste such as that defined in one of the preceding claims.

20. Use of a pigment paste such as that defined according to one of claims 1 to 18 for producing a lipstick.

## Patentansprüche

1. Wasserfreie Pigmentpaste, umfassend
- 20 bis 32 Vol.-% wenigstens eines festen Pigments, bezogen auf das gesamte Volumen der Pigmentpaste,
- ein Dispersionsmedium für das Pigment, das wenigstens ein wasserfreies Lösungsmittel, ausgewählt aus Kohlenwasserstoff, Ölen und Silikonölen, umfasst, und
- ein Dispergiermittel, das ein Polymer ist, das wenigstens eine Imid- oder Succinimidgruppe über eine kovalente Bindung an eine Polymerkette gebunden umfasst, kompatibel mit dem Dispersionsmedium.

2. Pigmentpaste nach Anspruch 1, **dadurch gekennzeichnet, dass** die Polymerkette ein Molekulargewicht von wenigstens 600 g hat.

3. Pigmentpaste nach Anspruch 1 oder 2, **dadurch gekennzeichnet, dass** die Polymerkette ein Molekulargewicht von 1000 bis 1500 g hat.

4. Pigmentpaste nach einem der vorangehenden Ansprüche, **dadurch gekennzeichnet, dass** die Polymerkette ein Polyolefin ist.

5. Pigmentpaste nach einem der vorangehenden Ansprüche, **dadurch gekennzeichnet, dass** die Polymerkette ein Polyisobutylen ist.

6. Pigmentpaste nach einem der vorangehenden Ansprüche, **dadurch gekennzeichnet, dass** das Dispergiermittel ein Polyisobutylensuccinimid ist.

7. Pigmentpaste nach einem der vorangehenden Ansprüche, **dadurch gekennzeichnet, dass** das Dispergiermittel ein Polymer ist, das durch Kondensation eines Amins an ein Bernsteinsäureanhydrid mit einer gesättigten oder ungesättigten Kohlenwasserstoffkette erhalten wird.

8. Pigmentpaste nach einem der vorangehenden Ansprüche, **dadurch gekennzeichnet, dass** die Massefraktion des Dispergiermittels in der Pigmentpaste 1 bis 10% der Gesamtmasse der Pigmentpaste ist und vorzugsweise in der Größenordnung von 5% liegt.

9. Pigmentpaste nach einem der vorangehenden Ansprüche, **dadurch gekennzeichnet, dass** das Pigment unter den Mineralpigmenten, den organischen Pigmenten, den Perlmuttpigmenten und deren Gemischen ausgewählt ist.

10. Pigmentpaste nach Anspruch 9, **dadurch gekennzeichnet, dass** die Mineralpigmente aus Titandioxid, Zinkdioxid, Redem Eisenoxid, Yellowem Eisenoxid, braunem Eisenoxid, schwarzem Eisenoxid und deren Gemischen ausgewählt sind.

11. Pigmentpaste nach Anspruch 9, **dadurch gekennzeichnet, dass** die organischen Pigmente aus reinen organischen Pigmenten oder Pigmenten des Lacktyps ausgewählt sind.

12. Pigmentpaste nach Anspruch 9 oder 11, **dadurch gekennzeichnet, dass** das organische Pigment D&C Red Nr. 36 ist.

13. Pigmentpaste nach Anspruch 9, **dadurch gekennzeichnet, dass** das Pigment ein Lack ist, ausgewählt aus organischen Lacken von Barium, Strontium, Calcium oder Aluminium, den Lacken auf der Grundlage von Cochenille-Carmin, D&C Red Nr. 7 Calcium Lake, D&C Red Nr. 27 Aluminium Lake, D&C Red Nr. 21 Aluminium Lake, FD&C Yellow Nr. 5 Aluminium Lake, FD&C Yellow Nr. 6 Aluminium Lake, D&C Red Nr. 7 und FD&C Blue Nr. 1 Aluminium Lake und deren Gemischen.

14. Pigmentpaste nach Anspruch 9, **dadurch gekennzeichnet, dass** die reinen Pigmente ausgewählt sind aus: Glimmer, bedeckt mit Titan oder Wismutoxichlorid, Glimmer-Titan mit Eisenoxiden, Glimmer-Titan mit Eisenblau, Glimmer-Titan mit Chromoxid und Glimmertitan mit einem organischen Pigment, wie es in einem der Ansprüche 11-13 definiert ist.

15. Pigmentpaste nach einem der vorangehenden Ansprüche, **dadurch gekennzeichnet, dass** das wasserfreie Lösungsmittel unter den apolaren Ölen ausgewählt ist.

16. Pigmentpaste nach einem der vorangehenden Ansprüche, **dadurch gekennzeichnet, dass** das wasserfreie Lösungsmittel ein apolares Kohlenwasserstofföl ist, das aus hydriertem Polyisobuten, Squalan, aliphatischen Kohlenwasserstoffen und ihren Gemischen ausgewählt ist.

17. Pigmentpaste nach einem der vorangehenden Ansprüche, **dadurch gekennzeichnet, dass** das wasserfreie Lösungsmittel ein Silikonöl ist, ausgewählt aus Polydimethylsiloxanen (PDMS) und Phenylsilikonen wie z.B. Phenyltrimethiconen, Vinyldimethiconen, Diphenyldimethyconen, Diphenylmethyldiphenyltrisiloxanen und 2-Phenylethyltrimethylsiloxysilikaten und deren Gemischen.

18. Pigmentpaste nach einem der vorangehenden Ansprüche, **dadurch gekennzeichnet, dass** sie eine Viskosität von 2 bis 25 Poises (0,2 bis 2,5 Pa.s), vorzugsweise von 10 Poises (1 Pa.s) oder weniger, am besten von 5 Poises (0,5 Pa.s) oder weniger hat, wobei die Viskosität bei Umgebungstemperatur (20 bis 25°C) und bei einem Scherwert von 200 s⁻¹ beurteilt wird.

19. Kosmetikzusammensetzung, die ein kosmetisch annehmbares Medium und eine Pigmentpaste, wie sie in einem der voranstehenden Ansprüche definiert ist, enthält.

20. Verwendung der Pigmentpaste, wie sie in einem der Ansprüche 1 bis 18 definiert ist, zur Herstellung von Lippenstiften.
